# EUROPEAN PATENT APPLICATION

(11) **EP 3 513 817 A1**
(43) Date of publication of application: **24.07.2019**
(21) Application number: 18275196.6
(22) Date of filing: 28.12.2018
(51) Int. Cl.: A61L 2/18, A61L 2/26

(54) **STERILIZATION TRAY**

(30) Priority: 29.12.2017 US 201715858381
(71) Applicant: Ethicon, Inc., Somerville, NJ 08876 (US)
(72) Inventor: RHODES, Samuel J., Cincinnati, OH Ohio 45202 (US)
(74) Representative: Small, Gary James

(57) **Abstract**

A sterilization tray is disclosed. The sterilization tray comprises a basin and a platter including a depression and a port disposed through a terminal end of the depression. The basin and the platter define a chamber and the port defines a passage into the chamber. An adapter may be connected to the port to facilitate connection of an endoscope thereto. The tray and the endoscope may be disposed in a vacuum chamber of a sterilizer and subject to a sterilization cycle. A pressure differential may be created between the vacuum chamber and the first chamber. A sterilant may be drawn through the insertion tube and into the first chamber. The sterilant may also be drawn through the insertion tube and into the vacuum chamber.

## Description

### CROSS-REFERENCE TO CO-PENDING APPLICATIONS

This application is a counterpart of U.S. Patent Application Nos. 15/828,654, filed December 1, 2017; 15/830,331, filed December 4, 2017; 15/834,233, filed December 7, 2017; 15/844,237, filed December 15, 2017; and [400369-20034], filed December 29, 2017, each of which are incorporated by reference in their entirety.

### FIELD

The subject matter disclosed herein relates to containers used for sterilizing medical instruments.

### BACKGROUND

Endoscopes are challenging to sterilize for various reasons. For example, because pressure within a lumen decreases from the lumen's inlet as a function of length and diameter, the pressure drop must be overcome to ensure that sterilant passes through the entire lumen and reaches all surfaces of the lumen. Further, lumens may collect debris or be blocked by fluids, such as rinse water.

A dry booster is a device that may be connected to a lumen of an elongate medical device, such as an endoscope. When subject to a sterilization process in which pressure changes are implemented, pressure differentials between the inside of a dry booster at one end of the lumen and a vacuum chamber at the other end of a lumen help pass a sterilant through the lumen, which assists in sterilizing the lumen.

### SUMMARY

A sterilization tray particularly suited for sterilizing an elongate medical device, such as an endoscope, is disclosed herein. The sterilization tray may comprise a basin, a platter including a depression, and a port disposed through a terminal end of the depression. The tray may also include a lid, which may include a vent hole. The basin and the platter define a chamber and the port defines a passage into the chamber. In some embodiments, the passage is a sole passage. An adapter may be connected to or through the port. The adapter may include a conduit therethrough.

In some embodiments, the depression may be a trench. The trench may have an arcuate, angular, or semi-circular profile having a first width or first radius. The trench may also have a curved configuration. A spacer disposed within the trench. The spacer may have an arcuate, angular, or semi-circular profile having a second width or second radius that is smaller than the first width or first radius.

In some embodiments, the basin and the platter may additionally define a second chamber that is sealed from the first chamber. The second chamber may be sealed from the first chamber with a gasket. A vent hole in the platter may define a passage to the second volume.

The sterilization tray may be used to perform the following method and variations. The sterilization tray may be provided. An insertion tube of an endoscope may be positioned in the depression. The insertion tube may be connected to the port. The sterilization tray with the endoscope disposed therein may be disposed in a vacuum chamber of a sterilizer, and then subject to a sterilization cycle. In those embodiments where an adapter is disposed through the port, the step of connecting the insertion tube to the port includes connecting the insertion tube to the adapter.

A pressure differential may be created between the vacuum chamber and the first chamber. A sterilant may be drawn through the insertion tube and into the first chamber. The sterilant may also be drawn through the insertion tube and into the vacuum chamber.

### BRIEF DESCRIPTION OF THE DRAWINGS

While the specification concludes with claims, which particularly point out and distinctly claim the subject matter described herein, it is believed the subject matter will be better understood from the following description of certain examples taken in conjunction with the accompanying drawings, in which like reference numerals identify the same elements and in which:
Figure 1 depicts a perspective view of a sterilization tray;
Figure 2 depicts a top view of a platter of the sterilization tray of Figure 1;
Figure 3A depicts a top portion of an exploded view of the sterilization tray of Figure 1;
Figure 3B depicts a bottom portion of the exploded view of the sterilization tray of Figure 1; and
Figure 4 depicts a perspective cross-section view of a platter and a basin of the sterilization tray of Figure 1, the cross-section taken about section line 4-4 of Figure 2.

### DETAILED DESCRIPTION

The following description sets forth certain illustrative examples of the claimed subject matter. Other examples, features, aspects, embodiments, and advantages of the technology should become apparent to those skilled in the art from the following description. Accordingly, the drawings and descriptions should be regarded as illustrative in nature.

Successful sterilization of an endoscope may require accounting for various challenges. Sterilization trays for endoscopes may be designed to increase the likelihood that an endoscope may be successfully sterilized during a sterilization procedure by addressing each of the following three sterilization challenges. First, endoscopes are cumbersome to handle. Second, endoscope tubes are typically made from polyurethane, which absorbs sterilants such as hydrogen peroxide. When one portion of an endoscope tube touches another portion of the endoscope tube, or any other material or object that absorbs sterilant, a so-called "mated surface" is formed, which effectively provides additional mass through which a sterilant must penetrate to achieve sterilization. Third, endoscopes include multiple long and narrow lumens that may restrict the flow of a sterilant therethrough.

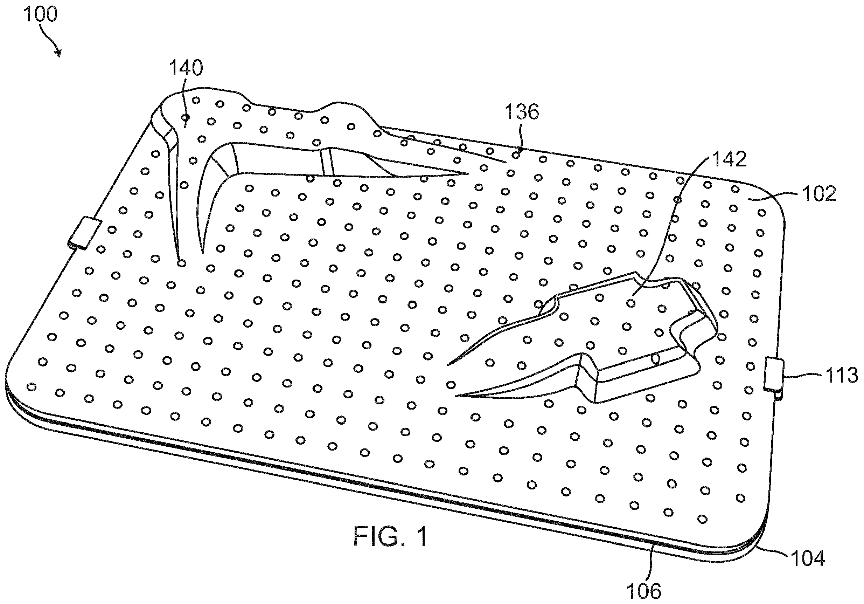

With reference to Figure 1, sterilization tray 100 includes a lid 102, a basin 104, and a platter 106. Clasps 113 may be used to secure together lid 102, basin 104, and platter 106. Lid 102 may be mated to basin 104 and platter 106 to shelter an endoscope therein. Lid 102 may include various vent holes or pores 136 therethrough to allow a gas (e.g., air or sterilant) to pass from the surrounding environment (e.g., vacuum chamber) and into the sterilization tray. Lid 102 may further include a first protrusion 140 that generally conforms to the shape of the endoscope's control body and a second protrusion 142 that conforms generally to the endoscope's light connector body.

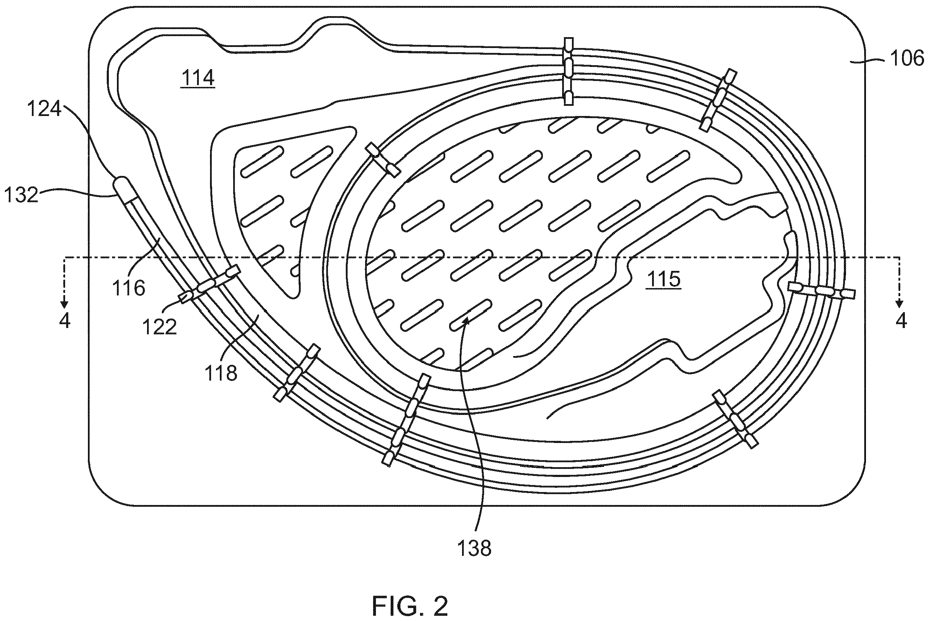

Figure 2 shows a top view of platter 106. Platter 106 may include a first depression 114, a second depression 115, a first trench 116, and a second trench 118. First depression 114 may conform generally to a shape of an endoscope's control body. Second depression 115 may conform generally to a shape of an endoscope's light-connector body. Trench 116 may be configured to conform generally to the shape of an endoscope's insertion tube. Trench 118 may be configured to conform generally to the shape of an endoscope's umbilical tube. Thus, trenches 116 and 118 may have an arcuate, angular, semi-circular, u-shaped, or similar profile that is slightly larger than an endoscope's tubes. In some embodiments, trenches 116 and 118 may have a coiled configuration to allow for disposing the insertion and umbilical tubes therein in a coiled configuration.

Depression inserts or spacers (not shown) may be disposed upon or within depressions 114 and 115, and trench inserts or spacers 122 may be disposed within trenches 116 and 118. These spacers may be utilized to avoid or minimize possible creation of mated surfaces between portions of an endoscope and portions of platter 106 by maintaining spacing between the endoscope and platter 106. Depression spacers and trench spacers 122 may be fabricated from a material through which a sterilant may readily pass, such that when the material contacts, e.g., a polyurethane endoscope tube, a so-called mated surface is not formed, or, if one is formed, the mate does not provide substantial additional challenge to a sterilant penetrating the mated surface. For example, the spacers may be fabricated from a silicone rubber.

The depression spacers may have the form of a sheet, and may include protrusions that are, e.g., conical or cylindrical in shape. Trench spacers 122 may have an internal profile that conforms closely to the endoscope's tubes such that the tubes may be secured therein. Accordingly, spacers 122 may have an arcuate, angular, semi-circular, or angled profile having a radius or width that is smaller than the radius or width trenches 116 and 118. Spacers 122 may be somewhat flexible to facilitate placement of the endoscope's tubes therein, while still providing a secure hold. In some embodiments, cutouts may be provided along trenches 116 and 118 into which spacers 122 may be secured. In other embodiments, spacers 122 may fit within trenches 116 and 118 by a friction fit.

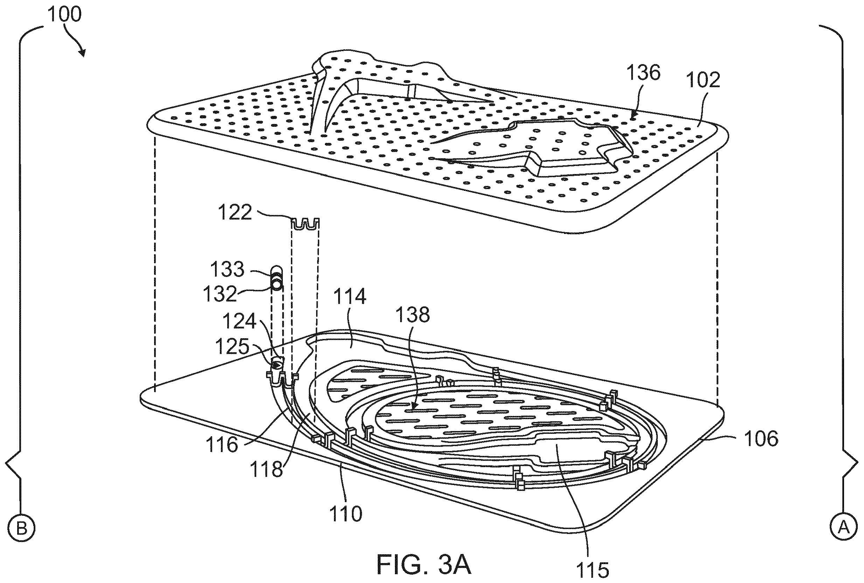

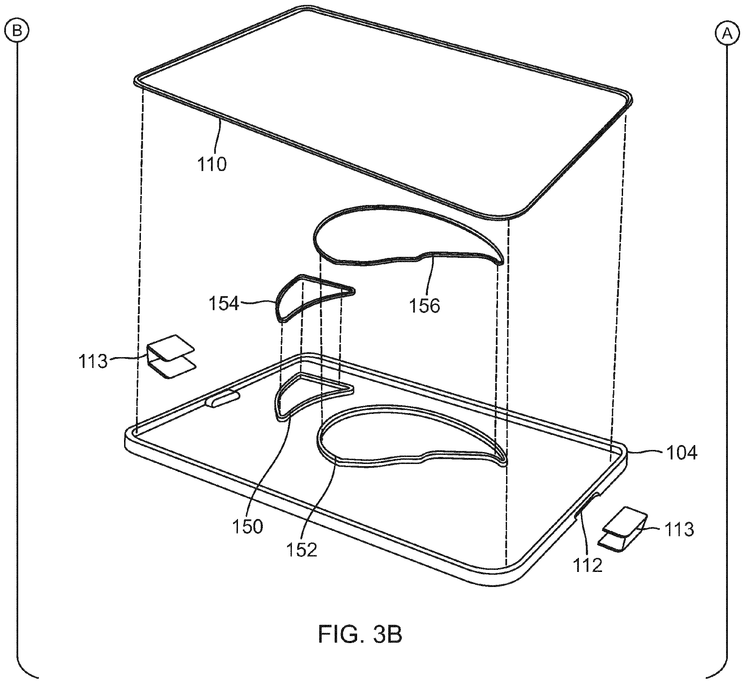

Figures 3A and 3B show an exploded view of tray 100. Basin 104 includes handles 112 to which clasps 113 may be mated. Basin 104 also includes a first enclosure 150 and a second enclosure 152, each projecting from the base of basin 104. Gasket 110 may be included and positioned proximate the outside top edges of basin 104 so that it may be squeezed between these top edges and the outside bottom edges of platter 106. Further, gasket 154 may be included and positioned proximate top edges of enclosure 150 such that it may be squeezed between these top edges and the bottom of platter 106. Similarly, gasket 156 may be included and positioned proximate to top edges of enclosure 152 such that it may be squeezed between these top edges and the bottom of platter 106. Gaskets 110, 154, and 156 may be made from silicone rubber.

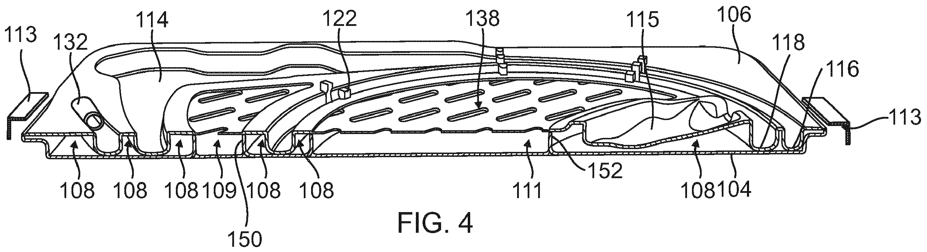

Figure 4 shows a cross section of platter 106 disposed atop basin 104. A first volume or chamber 108 may be defined by inner surfaces of basin 104 and platter 106. In some embodiments additional volumes or chambers may also be defined by various inner walls and surfaces of platter 106 and basin 104. For example, second volume or chamber 109 may be bound on its sides by enclosure 150 and third volume or chamber 111 may be bound on its sides by enclosure 152. Seals or gaskets 110, 154, and 156 help reduce the likelihood or eliminate the possibility of any gas (e.g., air or sterilant) from leaking out of or into volume 108, e.g., from volume 108 to outside tray 100, or from volume 109 into volume 108. Clasps 113 may help compress basin 104 and platter 106 upon gaskets 110, 154, and 156, further reducing the likelihood of leaks. Sealing volume 108 may be accomplished in manners excluding gaskets. For example, basin 104 and platter 106 may be permanently attached in a manner that prevents leaks, e.g., by gluing or ultrasonic welding.

Platter 106 may include a port that provides fluid access to volume 108. For example, first trench 116 may include a terminal surface 124 with a port 125 disposed therethrough. Thus, when platter 106 is mated to basin 104, the port functions as a passage for a fluid (e.g., air or gas, such as hydrogen peroxide) to pass into and out of chamber 108. Because platter 106 and basin 104 may be sealed together, e.g., with gaskets 110, 154, and 156, port 125 may function as the sole passage through which a gas may pass into and out of chamber 108. An adapter 132 having a conduit therethrough may be connected to the port to facilitate connection of an endoscope to chamber 108. For example, adapter 132 may be a tubing adapter or collar made from silicone rubber, which is configured to mate to a distal or free end of the endoscope's insertion tube. Adapter 132 may be bonded or glued to the port. Alternatively, adapter132 may further include a mating feature, such as groove 133, to maintain its position relative to the port and terminal end 124.

The free end of an endoscope's insertion tube may be connected to adapter 132, such that the endoscope's control body is fluidly connected to first volume or chamber 108 via lumens in the insertion tube. So connected, a gaseous sterilant may pass into the control body, through insertion tube, and into first volume or chamber 108. Therefore, during a sterilization cycle that includes pressure changes and that utilizes a gaseous sterilant, first volume or chamber 108 may function as a dry booster, which assists in drawing the sterilant trough the endoscope's lumens and removing the sterilant therefrom. Dry boosters are described in U.S. Patent Nos. 6,451,255 and 7,229,591, which are hereby incorporated by reference in their entirety. In short, dry boosters may be used to create a pressure differential between a so-called "booster" volume (here, first volume or chamber 108) and the vacuum chamber of a sterilizer to assist in introducing and removing sterilant through a lumen connecting the booster volume and the vacuum chamber. These references describe adapters for connecting an endoscope to a dry booster. Thus, adapter 132 may have the form of any of the dry-booster adapters described therein, or it may include any of the design elements therefrom.

Alternatively, instead of port 125 disposed in terminal end 124, one or more ports (not shown) may be disposed on platter 106 proximate to or within depression 114 to provide fluidic access to chamber 108. Tubing may be used to connect these tray ports to ports on the endoscope's control body, which provide access to lumens within the endoscope's insertion tube and/or umbilical tube. In these embodiments, air or another gas may be passed into the free end of the insertion tube, through the insertion tube, through the control body, through the tubing connecting the control-body port to the tray port, and into chamber 108. Additionally, in these embodiments, air or another gas may be pulled from chamber 108, through the tubing connecting the control-body port to the tray port, through the control body, through the insertion tube, and out of the free end of the insertion tube. Although the direction of flow through the endoscope is reversed as compared to those embodiments including port 125, chamber 108 acts as a booster volume in the same manner for both embodiments. Furthermore, in those embodiments that include multiple tray ports proximate or within depression 114, the tubes connecting these ports to the control-body ports may provide restrictions to the flow of a gas therethrough. Such flow restrictions may be implemented to further facilitate sterilization of the endoscope as is described in U.S. Patent Application No. 15/844,237, filed December 15, 2017, which is hereby incorporated by reference in its entirety.

The volume of chamber 108 may be chosen based on various design considerations. To achieve such a chosen volume, chambers 109 and 111 may be provided and tailored accordingly by changing the shapes of enclosures 150 and 152. However, because volumes 109 and 111 are not booster volumes, platter 106 should be designed to permit gasses to readily pass therein. For example, as shown in Figure 4, vent holes or pores 138 may be disposed through platter 106 above volumes 109 and 111, to provide this access.

Tray 100, with an endoscope disposed therein and an insertion tube positioned in trench 116 and connected to adapter 132, may be subject to a sterilization cycle within a vacuum chamber of a sterilizer, such as the STERRAD® System, STERRAD® NX System or STERRAD® 100NX System of Advanced Sterilization Products, Division of Ethicon US, LLC, a Johnson & Johnson company. As pressure is lowered within the vacuum chamber, the pressure within first volume or chamber 108 concomitantly drops, but lags behind the pressure in the vacuum chamber because of the time required to withdraw gas from volume or chamber 108, through the insertion tube. Similarly, when pressure within the vacuum chamber is raised, the pressure within first volume or chamber 108 concomitantly rises, but it lags behind the pressure in the vacuum chamber because of the time required to pass gas into volume or chamber 108, through the insertion tube. Accordingly, when pressure within the vacuum chamber is raised by introducing a gaseous sterilant into the vacuum chamber, the lower pressure within first volume or chamber 108 provides suction through the lumens of the insertion tube, which may assist sterilant penetrating throughout the lumens. Similarly, when pressure is lowered in the vacuum chamber, suction through the lumens may assist in flushing or removing any residual sterilant or moisture from the lumens.

Although not shown, in some embodiments, the sterilization tray may include a port at a terminal end of trench 118 that provides fluid communication to volume or chamber 108. In these embodiments, an endoscope's umbilical tube may be placed in fluid communication with volume or chamber 108 in a similar fashion to the endoscope's insertion tube.

Sterilization tray 100 may be used according to the following exemplary method. First, sterilization tray 100 may be provided. Second, lid 102 may be removed or unmated from platter 106 and/or basin 104. Third, an endoscope may be disposed within platter 106. Specifically, the endoscope's control body may be disposed in first depression 114, the endoscope's light-connector body may be disposed in second depression 115, the endoscope's insertion tube may be disposed within first trench 116, and the endoscope's umbilical tube may be disposed within second trench 118. Fourth, the distal end of the endoscope's insertion tube maybe connected to adapter 132. Fifth, lid 102 maybe mated to platter 106 and/or basin 104. Sixth, tray 100 may be placed within a vacuum chamber of a sterilization system. Seventh, a sterilization cycle may be commenced. Eighth, pressure in the vacuum chamber may be lowered. Ninth, a sterilant, e.g., hydrogen peroxide, may be introduced into the chamber. Tenth, because volume or chamber 108 functions as a dry-booster volume, the sterilant may be drawn through a lumen in the insertion tube and into volume or chamber 108 assisted by a pressure differential between volume or chamber 108 and the vacuum chamber. Eleventh, the vacuum chamber may be vented to raise the pressure and introduce another gas, e.g., air, therein. Because volume or chamber 108 functions as a dry-booster volume, the gas, e.g., air, may be drawn through the lumen in the insertion tube and into volume or chamber 108 assisted by a pressure differential between volume or chamber 108 and the vacuum chamber, which may assist in flushing the sterilant from the lumen. Twelfth, pressure in the vacuum chamber may be lowered. Because volume or chamber 108 functions as a dry-booster volume, gas within volume or chamber 108 may be drawn through the lumen in the insertion tube and into the vacuum chamber, which may further assist in flushing the sterilant from the lumen. Thirteenth, tray 100 may be removed from the vacuum chamber of the sterilization system.

It should be understood that any of the examples and/or embodiments described herein may include various other features in addition to or in lieu of those described above. The teachings, expressions, embodiments, examples, etc. described herein should not be viewed in isolation relative to each other. Various suitable ways in which the teachings herein may be combined should be readily apparent to those of ordinary skill in the art in view of the teachings herein.

Having shown and described exemplary embodiments of the subject matter contained herein, further adaptations of the methods and systems described herein may be accomplished by appropriate modifications without departing from the scope of the claims. Some such modifications should be apparent to those skilled in the art. For instance, the examples, embodiments, geometries, materials, dimensions, ratios, steps, and the like discussed above are illustrative. Accordingly, the claims should not be limited to the specific details of structure and operation set forth in the written description and drawings.

## Claims

1. A sterilization tray, comprising,
a basin; and
a platter including a depression and a port disposed through a terminal end of the depression;
wherein the basin and the platter define a chamber and the port defines a passage into the chamber.

2. The sterilization tray of claim 1, wherein the passage is a sole passage.

3. The sterilization tray of claim 1, wherein an adapter is connected to the port, the adapter including a conduit therethrough.

4. The sterilization tray of claim 3, wherein the depression is a trench, preferably wherein the trench has a semi-circular profile having a first radius, more preferably wherein the trench has a curved configuration, still more preferably further comprising a spacer disposed within the trench, the spacer having a semi-circular profile having a second radius that is smaller than the first radius.

5. The sterilization tray of claim 1, wherein the basin and the platter further define a second chamber that is sealed from the first chamber

6. The sterilization tray of claim 5, wherein the second chamber is sealed from the first chamber with a gasket.

7. The sterilization tray of claim 6, wherein the platter further includes a first vent hole defining a passage to the second volume.

8. The sterilization tray of claim 7, further comprising a lid.

9. The sterilization tray of claim 8, wherein the lid includes a second vent hole therethrough.

10. A method of sterilizing an endoscope, comprising:
providing a sterilization tray, the sterilization tray including
a basin, and
a platter including a depression and a port disposed through a terminal end of the depression,
wherein the basin and the platter define a chamber and the port defines a passage into the chamber;
positioning an insertion tube of an endoscope in the depression;
connecting the insertion tube to the port;
positioning the sterilization tray with the endoscope disposed therein in a vacuum chamber of a sterilizer; and
subjecting the sterilization tray with the endoscope disposed therein to a sterilization cycle.

11. The method of claim 10, wherein the sterilization tray further includes an adapter disposed through the port, and wherein the step of connecting the insertion tube to the port includes connecting the insertion tube to the adapter.

12. The method of claim 11, further comprising creating a pressure differential between the vacuum chamber and the first chamber.

13. The method of claim 12, further comprising drawing a sterilant through the insertion tube and into the first chamber.

14. The method of claim 13, further comprising drawing the sterilant through the insertion tube and into the vacuum chamber.

15. The method of claim 14, wherein the sterilization tray further includes a second chamber sealed from the first chamber.
